# DEMANDE DE BREVET EUROPEEN

(11) **EP 1 038 511 A1**
(43) Date de publication de la demande: **27.09.2000**
(21) Numéro de dépôt: 00490010.6
(22) Date de dépôt: 29.02.2000
(51) Int. Cl.: A61F 13/56, A61F 13/58

(54) **Couche-culotte à fermeture mécanique et adhésive**

(30) Priorité: 22.03.1999 FR 9903694
(71) Demandeur: Proteco, 59494 Petite Forêt (FR)
(72) Inventeur: Brutin, Jean-Marc, 59780 Camphin en Pevele (FR)
(74) Mandataire: Hénnion, Jean-Claude

(57) **Abrégé**

La couche-culotte (1) de l'invention est formée d'une feuille extérieure imperméable (2), d'une feuille intérieure perméable (3), d'un matelas absorbant interposé entre les deux dites feuilles (2,3) et d'un système d'attache permettant de réaliser la fermeture de la couche (8). Le système d'attache comporte selon un côté, droit (8) ou gauche (9), de la couche-culotte (1) des moyens d'attache mécaniques du type auto-agrippant (10,12); et selon l'autre côté , gauche (9) ou droit (8), des moyens d'attache adhésifs (11,13).

## Description

La présente invention concerne le domaine des articles d'hygiène jetables, que ce soit les couches-culottes pour bébés ou les articles pour incontinence adulte, l'ensemble de ces articles étant dénommés dans la suite du présent texte par simplification sous le terme couche-culotte.

Une couche-culotte est formée d'une feuille extérieure imperméable, d'une feuille intérieure perméable, d'un matelas absorbant interposé entre les deux dites feuilles et d'un système d'attache permettant de réaliser la fermeture de la couche ; ce système consiste dans des premiers moyens d'attache disposés latéralement selon l'extrémité de la couche constituant la partie arrière et des seconds moyens d'attache disposés selon l'autre extrémité de la couche formant la partie avant de celle-ci.

Les premiers moyens d'attache, jusqu'à une époque récente, ont consisté exclusivement en des moyens d'attache adhésifs. Il s'agit plus particulièrement de pattes d'attache fixées sur la feuille extérieure imperméable selon les bords opposés de l'extrémité constituant la partie arrière de la couche. Chaque patte d'attache comprend un ruban dont une face est enduite de colle sensible à la pression. Dans ce cas, les seconds moyens d'attache sont constitués par une zone ou un élément de renfort disposé sur la surface externe ou interne de la feuille imperméable au niveau de l'autre extrémité de la couche-culotte, constituant la partie avant. Lors de la mise en place de la couche sur le corps de l'utilisateur, pour réaliser la fermeture de celle-ci, il suffit d'appliquer la partie adhésive de la patte d'attache sur la zone ou l'élément de renfort disposé de manière frontale sur la partie avant.

Parmi les modes de réalisation possibles, on peut citer la mise en place, comme élément de renfort, d'une bande frontale en matière plastique, collée transversalement sur la partie avant de la couche-culotte, extérieurement à la feuille imperméable. La patte d'attache adhésive est appliquée sur cette bande en matière plastique pour réaliser la fermeture de la couche. Il est possible d'enlever la patte latérale adhésive pour vérifier l'état de la couche puis refixer celle-ci en l'appliquant de nouveau sur la bande frontale. Dans l'exemple de réalisation du document FR-A-2 516 757, cette bande frontale a une surface lisse tandis que dans le document FR-A- 2 534781, elle a une surface comportant des empreintes ou des bossages.

La capacité de refixation de la patte d'attache adhésive peut être amoindrie ou annulée en cas de salissure ou de pollution du revêtement adhésif. Or, s'agissant de couche-culotte pour bébé, cette vérification se fait généralement lorsque l'opérateur manipule des poudres ou des corps gras pour le nettoyage ou l'entretien de la peau du bébé. Si ces poudres ou corps gras viennent en contact avec le revêtement adhésif porté par la patte d'attache, soit la refixation est impossible, soit elle peut être perturbée au point de la rendre inefficace et d'occasionner la réouverture de la couche à l'occasion des mouvements du bébé.

Pour pallier cet inconvénient majeur, il a été proposé d'utiliser des moyens d'attache mécaniques du type auto-agrippant, bien connus dans leurs applications liées au textile et à l'habillement sous la marque Velcro. Ces moyens comportent des éléments mâles du type crochet et des éléments femelles du type boucle. Dans le cas de la couche-culotte, les éléments mâles sont généralement portés par les pattes d'attaches latérales fixées selon les bords opposés de la partie arrière de la couche, tandis que les éléments femelles sont portés de manière frontale sur la partie avant de la couche. La pollution éventuelle par des poudres ou des corps gras, des crochets et/ou des boucles n'est en rien gênante lors de la fermeture de la couche, n'empêchant pas la pénétration et l'imbrication des crochets dans les boucles.

Il est indéniable que la mise en oeuvre d'éléments d'attache mécanique permet de résoudre totalement le problème technique lié aux salissures ou à la pollution des éléments d'attache adhésif. Le seul véritable inconvénient réside dans le prix de revient d'une telle solution, même si des progrès très importants ont été faits dans la fabrication d'éléments mâles et d'éléments femelles destinés uniquement à un usage dit jetable, avec possibilité d'ouverture et de refermeture limitée à 3 ou 4 fois.

Néanmoins, le surcoût entraîné par la mise en oeuvre d'éléments d'attache mécaniques se révèle un obstacle majeur en ce qui concerne sa généralisation à l'ensemble du marché des couches-culottes jetables.

Le but que s'est fixé le demandeur est de proposer une couche-culotte du type précité dont le coût est inférieur à une couche mettant en oeuvre de manière classique des moyens de fermeture d'attache mécaniques tout en ne présentant pas l'inconvénient dû à la salissure ou la pollution par des poudres ou corps gras.

Ce but est parfaitement atteint par la couche-culotte de l'invention qui, de manière caractéristique, comporte selon un côté, droit ou gauche, des moyens d'attache mécaniques et selon l'autre côté, gauche ou droit, des moyens d'attache adhésifs. Le demandeur est parti du constat que la vérification de l'état de la couche pouvait être faite en n'enlevant les moyens d'attache que d'un seul côté. Ce sont donc les moyens d'attache de ce seul côté qui seront des moyens d'attache mécaniques, en sorte que la salissure ou la pollution par des poudres ou des corps gras n'entraînera aucune gêne pour la refixation ultérieure. Par contre, l'autre côté peut être, sans préjudice, pourvu de moyens d'attache adhésifs, puisque ceux-ci n'auront pas vocation à être enlevés pour être refixés, à l'occasion des vérifications.

De préférence, les moyens d'attache mécaniques comportent, sur la face visible, lorsque la couche-culotte est placée sur l'utilisateur, un signe distinctif caractéristique. Ce signe distinctif a pour but de signaler à l'opérateur que c'est de ce côté qu'il doit préférentiellement effectuer l'enlèvement des moyens d'attache pour la vérification de l'état de la couche.

De même, les moyens d'attache adhésifs peuvent comporter, sur la face visible, lorsque la couche est placée sur l'utilisateur, un signe distinctif caractéristique, distinct de celui porté par les moyens d'attache mécaniques.

Par exemple, ce signe peut être constitué par une pastille ronde de couleur verte sur les moyens d'attache mécaniques et une pastille ronde de couleur rouge sur les moyens d'attache adhésifs, par analogie avec les feux tricolores de circulation.

Selon un mode préféré de réalisation, les moyens d'attache comportent des pattes latérales fixées selon les bords opposés de la partie arrière de la couche. De manière caractéristique selon l'invention, selon un premier côté, une première patte d'attache latérale porte des éléments mâles du type crochets (ou femelles du type boucles) et selon le second côté, une seconde patte d'attache latérale porte un revêtement adhésif ; de plus, dans ce cas, le renfort frontal est constitué selon le premier côté par une bande portant des éléments femelles du type à boucles (ou mâles du type crochets) aptes à coopérer avec les crochets (ou avec les boucles) de la première patte latérale et selon le second côté par une bande de renfort fixée de manière externe ou interne sur la feuille extérieure imperméable de la couche. Avantageusement, selon ce mode préféré de réalisation, la bande frontale est une bande unique, apte à être placée en une seule opération sur la partie avant de la couche, et constituée d'une bande de matière plastique sur une portion de laquelle ont été rapportés des éléments femelles du type à boucles (ou mâles du type crochets).

La présente invention sera mieux comprise à la lecture de la description qui va être faite d'un mode préféré de réalisation d'une couche-culotte à moyens d'attaches mécaniques et adhésifs, illustré par le dessin annexé dans lequel :
- les figures 1 et 2 sont des représentations schématiques en plan d'une couche-culotte,à l'état déplié, face extérieure pour la figure 1 et face intérieure pour la figure 2 et,
- la figure 3 est une représentation schématique en perspective d'une couche-culotte à l'état fermé.

La couche-culotte 1 est formée d'une feuille extérieure imperméable 2, d'une feuille intérieure perméable 3, d'un matelas absorbant interposé entre les deux dites feuilles 2, 3 et d'un système d'attache permettant de réaliser la fermeture de la couche autour du corps de l'utilisateur.

Par souci de simplification, sur les figures annexées, n'ont pas été représentés ni le matelas absorbant, ni les différents constituants qui peuvent également entrer dans la composition d'une couche-culotte, à savoir l'élastiquage permettant d'obtenir une fermeture sensiblement étanche autour de la jambe, les barrières fécales, disposées de chaque côté du matelas absorbant, entre celui-ci et le système d'élastiquage, et enfin le système de ceinture élastique, le long des bords transversaux opposés de la couche.

Conformément à l'invention, le système d'attache, permettant de réaliser la fermeture de la couche 1 sur elle-même, est constitué selon un côté, droit ou gauche de la couche, par des moyens d'attache mécaniques et selon l'autre côté, gauche ou droit de la couche, par des moyens d'attache adhésifs.

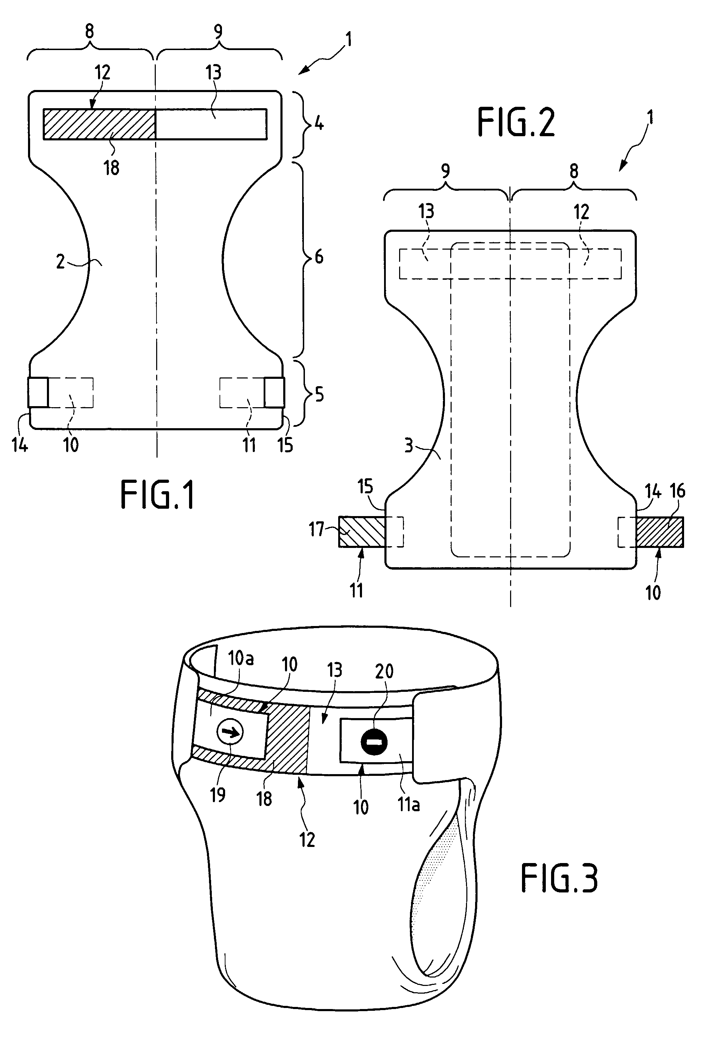

Dans l'exemple illustré, la couche-culotte 1 a une forme en sablier avec des parties avant 4 et arrière 5 qui ont une largeur plus importante que la partie centrale 6 d'entrejambe.3 Les termes avant et arrière sont pris en regard du positionnement desdites parties relativement au corps de l'utilisateur. Les moyens d'attache mécanique sont disposés selon le côté gauche 8 de la couche 1 tandis que les moyens d'attache adhésifs sont disposés selon le côté droit 9 de la couche 1. Les moyens d'attache sont, dans l'exemple illustré, constitués d'une part par une patte latérale 10, 11 et d'autre part par une bande frontale 12, 13. Les deux pattes latérales 10, 11 sont fixées selon les deux bords opposés 14, 15 de la partie arrière 5 de la couche 1. Sur la figure 1, les deux pattes d'attache latérale 10, 11 sont montrées à l'état replié, selon les deux bords 14, 15 tandis que sur la figure 2 elles sont montrées à l'état déployé.

La patte d'attache mécanique 10 comporte des crochets 16, tandis que la patte d'attache adhésive comporte un revêtement adhésif sensible à la pression 17.

Les deux bandes frontales 12, 13 sont disposées selon la partie avant 4 de la couche 1. La bande frontale 12, correspondant au moyen d'attache mécanique, comporte des boucles 18 aptes à coopérer avec les crochets 16 portés par la patte d'attache latérale 10. La bande frontale 13, correspondant au moyen d'attache adhésif, est constituée par une bande de matière plastique qui est soit collée extérieurement sur la feuille imperméable 2, soit collée intérieurement sur cette même feuille imperméable 2, de manière à constituer une zone de renforcement sur laquelle est appliqué le revêtement adhésif 17 de la patte d'attache latérale 11.

Dans le mode de réalisation illustré, les deux bande frontales 12, 13 sont dans le prolongement l'une de l'autre et occupent quasiment toute la largeur de la partie avant 4 de la couche 1. Il peut d'ailleurs s'agir d'une même bande de matière plastique ayant cette largeur totale et sur une portion de laquelle ont été rapportés, notamment par collage, les éléments à boucles 18, aptes à coopérer avec les crochets 16 de la patte d'attache 10. Il n'y a cependant pas une obligation absolue à ce que des éléments à boucle 18 soient disposés sur toute la partie gauche 8 de la couche sur la bande frontale correspondante. Les éléments à boucles 18 peuvent ne recouvrir que la zone de ladite bande frontale 12 nécessaire au réglage de la couche 1 lors du positionnement autour du corps de l'utilisateur.

La mise en place de la couche 1 est effectuée dans des conditions habituelles. L'opérateur referme la couche 1 autour du corps de l'utilisateur en utilisant, dans l'ordre qui lui convient, la patte d'attache mécanique 10 et la patte d'attache adhésive 11. Par contre, lors de la vérification de l'état de la couche, l'opérateur sera amené à enlever préférentiellement la patte d'attache mécanique 10 de manière à permettre l'ouverture partielle de la couche, tout en laissant en place la patte d'attache adhésive 11. Etant donné que la patte d'attache adhésive 11 ne sera pas enlevée lors de cette vérification, il n'y a aucun risque qu'elle soit souillée ou polluée. Quant à la patte d'attache mécanique 10, la présence de poudre ou de corps gras sur les crochets 16 n'altère en rien son efficacité.

Pour inciter l'opérateur à ne se servir que de la patte d'attache mécanique 10, une information adéquate sera portée sur l'emballage des couches 1. L'opérateur pourra également facilement repérer de quel côté se trouve la patte d'attache mécanique 10, du fait de la présence des éléments à boucles 18 sur la bande frontale 12. Cependant, afin d'orienter encore plus précisément son choix, la patte d'attache mécanique 10, comporte sur la face visible 10a, lorsque la couche 1 est placée sur le corps de l'utilisateur, c'est-à-dire la face opposée à celle où se trouvent les crochets 13, un signe distinctif caractéristique 19. Ce signe 19 peut par analogie avec les feux tricolores de circulation, être une pastille ronde de couleur verte. Sur la patte d'attache adhésive 11 peut être disposée, par opposition, une pastille ronde de couleur rouge. Il peut s'agir bien sûr d'autres signes caractéristiques incitant l'opérateur à choisir la patte d'attache mécanique 10 et à délaisser la patte d'attache adhésive 11, par exemple par analogie avec les panneaux de signalisation du code de la route, la flèche de voie à sens unique pour la patte d'attache mécanique 10 et le sens interdit 20 pour la patte d'attache adhésive 11.

La présente invention n'est pas limitée au mode de réalisation qui a été décrit à titre d'exemple non exhaustif. En particulier, les pattes latérales d'attache peuvent ne pas être des éléments rapportés selon les bords opposés de la partie arrière 5 de la couche 1 mais être intégrés dans cette partie arrière 5 au niveau des deux extrémités latérales. De même la disposition des crochets et des boucles dans les moyens d'attache mécanique peut être inversée.

## Revendications

1. Couche-culotte (1) formée d'une feuille extérieure imperméable (2), d'une feuille intérieure perméable (3), d'un matelas absorbant interposé entre les deux dites feuilles (2, 3) et d'un système d'attache permettant de réaliser la fermeture de la couche caractérisée en ce que le système d'attache comporte selon un côté, droit ou gauche, de la couche-culotte (1) des moyens d'attache mécaniques du type auto-agrippant, et selon l'autre côté, gauche ou droit, des moyens d'attache adhésifs.

2. Couche-culotte selon la revendication 1 caractérisée en ce que les moyens d'attache mécaniques comportent, sur la face visible (10a), lorsque la couche-culotte (1) est placée sur l'utilisateur, un signe distinctif caractéristique (19).

3. Couche-culotte selon la revendication 2 caractérisée en ce que les moyens d'attache adhésifs comportent, sur la face visible (11a), lorsque la couche (1) est placée sur l'utilisateur, un signe distinctif caractéristique (20), distinct de celui porté par les moyens d'attache mécaniques.

4. Couche-culotte selon l'une des revendications 1 à 3 caractérisée en ce que les moyens d'attache comportant des pattes latérales (10, 11) fixées selon les bords opposés (14, 15) de la partie arrière de la couche (1), selon un premier côté (8), une première patte d'attache latérale (10) porte des crochets (16) (ou des boucles) et selon le second côté (9), une seconde patte d'attache latérale (11) porte un revêtement adhésif et en ce qu'un renfort frontal est constitué selon le premier côté (8) par une bande (12) portant des éléments femelles à boucles (18) (ou des éléments mâles de type crochets) aptes à coopérer avec les crochets (16) (ou avec les boucles) de la première patte latérale (10) et selon le second côté (9) par une bande (13) de renfort fixée de manière externe ou interne sur la feuille extérieure imperméable (2) de la couche (1).

5. Couche-culotte selon la revendication 4 caractérisée en ce que le renfort frontal est une bande unique, apte à être placée en une seule opération sur la partie avant (4) de la couche (1), et constituée d'une bande de matière plastique sur une portion de laquelle ont été rapportés des éléments femelles à boucles (8) (ou des éléments mâles du type crochets).
